# EUROPÄISCHE PATENTSCHRIFT

(11) **EP 1 248 562 B1**
(45) Veröffentlichungstag und Bekanntmachung des Hinweises auf die Patenterteilung: **08.12.2004**
(21) Anmeldenummer: 01914996.2
(22) Anmeldetag: 18.01.2001
(51) Int. Cl.: A61B 5/053

(54) **MESSANORDNUNG ZUR UNTERSUCHUNG EINES GEWEBEABSCHNITTS EINES PATIENTEN**
MEASUREMENT SYSTEM FOR EXAMINING A SECTION OF TISSUE ON A PATIENT
DISPOSITIF DE MESURE SERVANT A ANALYSER UNE PARTIE TISSULAIRE D'UN PATIENT

(30) Priorität: 18.01.2000 DE 10001825
(43) Veröffentlichungstag der Anmeldung: 16.10.2002
(73) Patentinhaber: SIEMENS AKTIENGESELLSCHAFT, 80333 München (DE)
(72) Erfinder: MERTELMEIER, Thomas, 91058 Erlangen (DE); SCHOLZ, Bernhard, 91336 Heroldsbach (DE)
(86) Internationale Anmeldenummer: PCT/DE2001/000213
(87) Internationale Veröffentlichungsnummer: WO 2001/052733

(56) Entgegenhaltungen:
- EP-A- 0 945 103
- WO-A-96/12439
- US-A- 4 291 708
- US-A- 5 143 079
- PIPERNO G ET AL: "BREAST CANCER SCREENING BY IMPEDANCE MEASUREMENTS" FRONTIERS OF MEDICAL AND BIOLOGICAL ENGINEERING,NL,VSP. ZEIST, Bd. 2, Nr. 2, 1990, Seiten 111-117, XP000676951 ISSN: 0921-3775

## Beschreibung

Die Erfindung betrifft eine Messanordnung zur Untersuchung eines Gewebeabschnittes eines Patienten, aufweisend ein Elektrodenarray, eine Signalquelle und eine Messschaltung, wobei die Signalquelle über wenigstens eine Elektrode des Elektrodenarrays dem Patienten elektrische Ströme und/oder Spannungen einprägt, welche die Messschaltung an mindestens einer Elektrode des Elektrodenarrays misst, und wobei das Elektrodenarray zumindest bereichsweise von einem elektrischen Leiterelement umgeben ist.

Als Stand der Technik sind z.B. aus dem SIEMENS-Prospekt "TransScan TS 2000" (interne Nr. BKW 62014 WS 10994) und der US-A-4 291 708 Messanordnungen zur Untersuchung von Gewebeabschnitten eines Patienten bekannt, bei denen das Elektrodenarray von einem elektrischen Leiterelement in Form eines Metallstreifens ("Guardring") umgeben ist, wobei der Metallstreifen und die Elektroden des Elektrodenarrays mit identischem Potential betrieben werden. Hierdurch wird zwar die Nachweisbarkeit von Leitfähigkeitsinhomogenitäten des untersuchten Gewebeabschnitts verbessert, es treten jedoch unerwünschte Signalerhöhungen an den Bildrändern des Impedanzbildes auf.

Im Zusammenhang mit dem Problem derartiger Randeffekte ist es aus der US-A-5 143 079 bekannt, Elektroden anderer, beispielsweise hexagonaler Form zu verwenden.

Der Erfindung liegt die Aufgabe zugrunde, eine Messanordnung der eingangs genannten Art so auszubilden, dass die erwähnten unerwünschte Randeffekte möglichst weitgehend oder völlig vermieden sind.

Die Aufgabe wird für die Messanordnung durch die Merkmale des Patentanspruchs 1 gelöst. Vorteilhafte Ausführungsformen der Messanordnung werden in den Unteransprüchen 2 bis 17 beschrieben.

Bei der erfindungsgemäßen Messanordnung liegt also das das Elektrodenarray umgebende Leiterelement auf einem vom Potential der Elektroden des Elektrodenarrays abweichenden Potential, das vorzugsweise unterhalb des Potentials der Elektroden des Elektrodenarrays liegt.

Insbesondere wenn das Leiterelement ein niedrigeres Potential als die Elektroden aufweist, werden die unerwünschten Randeffekte in den Impedanzbildern zumindest abgemildert und häufig völlig beseitigt. Da somit die überhöhten Signalwerte an den Bildrändern der Impedanzbilder zumindest vermindert werden, ergibt sich eine verbesserte Bildqualität der Impedanzbilder, d.h. Leitfähigkeitsinhomogenitäten zum Beispiel aufgrund vorhandener Karzinome oder Tumore können besser erkannt bzw. detektiert werden.

Bei dem Leiterelement kann es sich um ein Metallelement oder Kunststoffelement aus einem leitenden Kunststoffmaterial handeln, das in seiner Geometrie, insbesondere seiner Breite, an die jeweilige Anwendung der Messanordnung angepasst werden kann, z.B. im Falle der Mammadiagnostik an Brüste unterschiedlicher Größe.

In einer vorteilhaften Ausführungsform der Messanordnung wird eine weitere Verbesserung der Bildqualität durch Anbringung mindestens eines weiteren Leiterelements erreicht, welches vorteilhafterweise ebenfalls ein Potential aufweist, das vom Potential der Elektroden des Elektrodenarrays abweicht, wobei gemäß einer Variante der Erfindung die einzelnen Leiterelemente mit jeweils unterschiedlichen Potentialen beaufschlagt werden, so dass ein gewünschter Potentialverlauf über die Gesamtbreite sämtlicher aktiver Leiterelemente erzielt werden kann. Es können dann abhängig von Art, Größe und Form des zu untersuchenden Gewebeabschnitts bei der Untersuchung Potential und Anzahl der verwendeten Leiterelemente individuell gewählt werden, um eine ausreichende Unterdrückung unerwünschter Randeffekte zu erzielen.

Nach einer weiteren besonders vorteilhaften Ausführungsform sind die Elektroden der Messanordnung von einem inneren und äußeren Leiterelement umgeben. Dabei werden die Elektroden mit dem Potential 0 mV, das innere Leiterelement in einem Potentialbereich unterhalb von 0 mV bis - 5 mV und das äußere Leiterelement mit einem Potential von - 15 mV betrieben. Dabei wird das Potential des inneren Leiterelements in Abhängigkeit von dem jeweiligen Potential des äußeren Leiterelements, sei es automatisch oder durch eine Bedienperson, derart eingestellt, dass eine ausreichende Unterdrückung der unerwünschten Randeffekte der Impedanzbilder vorliegt.

Wenn mehrere Leiterelemente vorgesehen sind kann die jeweilige Potentialzuweisung und/oder Aktivierung einer bestimmten Anzahl von Leiterelementen bzw. die Aktivierung bestimmter Leiterelemente anhand von bestimmten Voreinstellungen erfolgen, welche sich aus der Erfahrung als günstig für den gerade vorliegenden zu untersuchenden Gewebeabschnitt erwiesen haben. Derartige Voreinstellungen können zunächst automatisch erfolgen und dann von einer Bedienperson abgewandelt und an den konkreten vorliegenden Gewebeabschnitt angepasst werden, um eine besonders hohe Bildqualität zu erreichen.

Falls mehrere Leiterelemente vorgesehen sind, können diese geometrisch unterschiedlich ausgebildet sein und insbesondere eine unterschiedliche Breite aufweisen.

Das Leiterelement bzw. die Leiterelemente können das Elektrodenarray bereichsweise oder vollständig umgeben. Es können auch lokale Leiterelemente vorgesehen sein, welche z.B. in Eckbereichen des Elektrodenarrays angeordnet sind, um dort verstärkt auftretenden unerwünschten Randeffekten entgegen zu wirken.

Dabei werden die lokalen Leiterelemente gemäß einer Variante der Erfindung mit einem Potential beaufschlagt, das von dem Potential eines Leiterelementes und dem Potential der Elektroden abweicht. Auch bezüglich der lokalen Leiterelemente kann die jeweilige Potentialzuweisung und/oder Aktivierung einer bestimmten Anzahl von lokalen Leiterelementen bzw. die Aktivierung bestimmter lokaler Leiterelemente anhand von bestimmten Voreinstellungen erfolgen, welche sich aus der Erfahrung als günstig für den jeweils zu untersuchenden Gewebeabschnitt erwiesen haben, wobei sich an eine zunächst automatisch erfolgende Einstellung eine individuelle Anpassung durch eine Bedienperson anschließen kann.

Gemäß einer weiteren vorteilhaften Ausführungsform kann mindestens ein Leiterelement aus einem Material mit begrenzter Leitfähigkeit (einem Widerstandsmaterial, z.B. einer an sich bekannten Widerstandslegierung wie Chromnickel, Konstantan etc.) zur Ausbildung eines Spannungsgradienten bestehen. Hierdurch entstehen z.B. über die Breite des Leiterelements abnehmende, zunehmende oder sonstige gewünschte Potentialverläufe, indem das Leiterelement an seinen Rändern mit unterschiedlichen Potentialen beaufschlagt wird.

Die Verwendung der Messanordnung besteht im Feststellen von Tumoren in lebendem menschlichem Gewebe, insbesondere in der Mammadiagnostik zum Feststellen von Tumoren in lebendem menschlichem Brustgewebe.

Weitere Einzelheiten der Erfindung gehen aus nachfolgend erläuterten, in den Zeichnungen dargestellten Ausführungsbeispielen der Erfindung hervor. Es zeigen:
- Fig. 1: in grob schematischer, teilweise blockschaltbildartiger Darstellung eine erfindungsgemäße Messanordnung,
- Fig. 2: in schematischer Darstellung die Elektrodenanordnung des Gerätes gemäß Fig. 1,
- Fig. 3: ein Impedanzbild, welches mit einer Messanordnung nach dem Stand der Technik aufgenommen ist,
- Fig. 4: ein mit der erfindungsgemäßen Messanordnung aufgenommenes Impedanzbild, und
- Fig. 5: in zu der Fig. 2 analoger Darstellung einen Ausschnitt der Elektrodenanordnung einer Variante der erfindungsgemäßen Messanordnung.

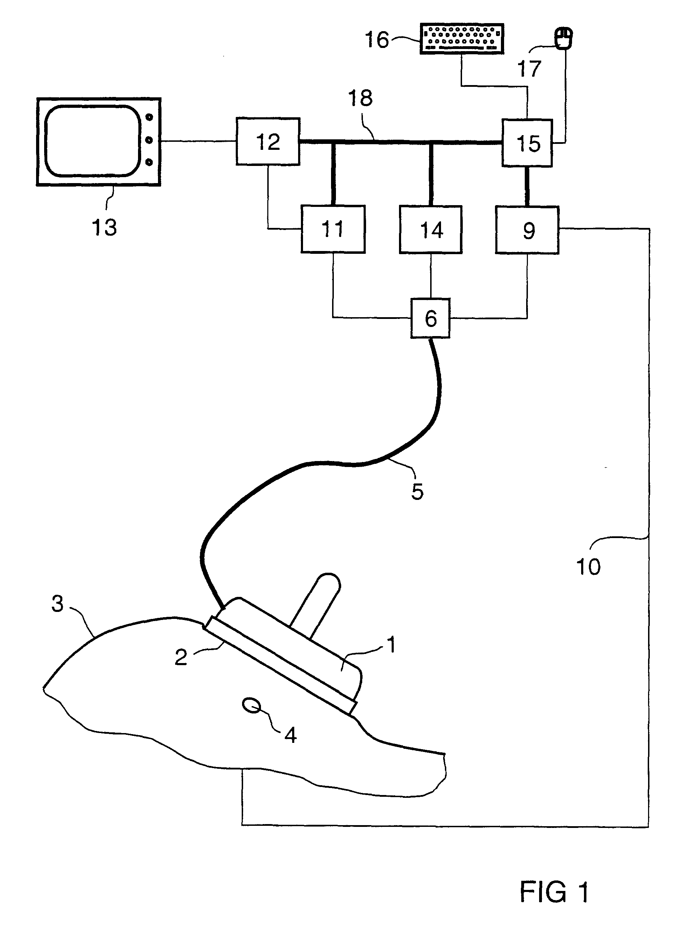

Die Fig. 1 zeigt eine erfindungsgemäße Messanordnung, die im Falle des dargestellten Anwendungsfalls dazu verwendet wird, einen Tumor in lebendenden menschlichen Gewebe festzustellen, wobei ein Anwendungsfall aus der Mammadiagnostik veranschaulicht ist, bei dem es darum geht, Tumore im lebenden menschlichen Brustgewebe zu erkennen.

Die Messanordnung weist einen handgehaltenen Applikator 1 auf, dessen Applikationsfläche 2 von der Bedienperson mit der Oberfläche desjenigen Gewebeabschnitts, also einer Mamma 3, in Berührung gebracht wird, indem ein Tumor 4 vermutet wird.

Der Applikator 1 weist an seiner Applikationsfläche 2 eine noch zu beschreibende Elektrodenanordnung auf, deren Elektroden und Leiterelemente über ein vieladriges Kabel 5 mit einer Interfaceschaltung 6 in Verbindung stehen.

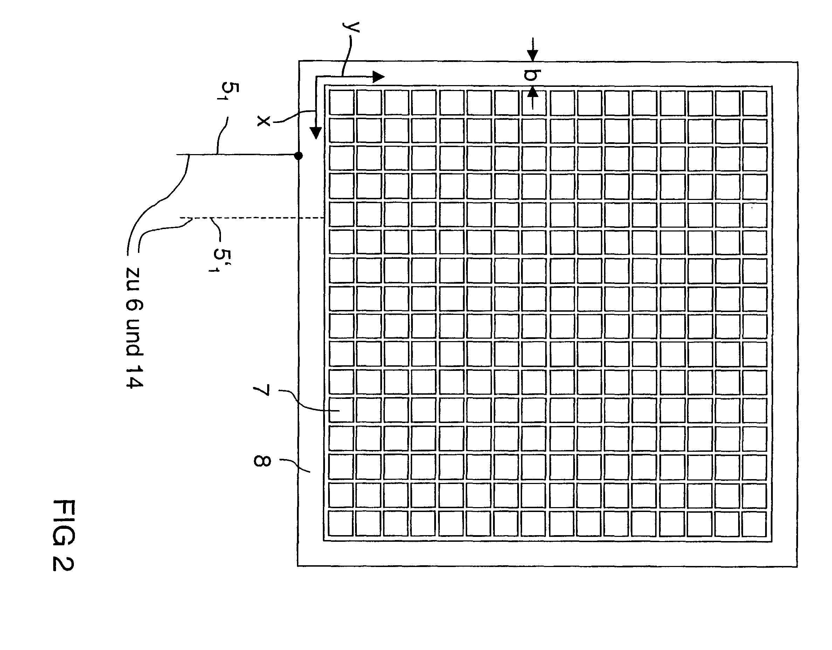

Die Elektrodenanordnung ist in Fig. 2 dargestellt und weist im Falle des beschriebenen Ausführungsbeispiels ein Elektrodenarray mit matrixartig angeordneten, von voneinander elektrisch isolierten, im Folgenden als Einzelelektroden 7 bezeichneten quadratischen Elektroden auf, das von einem rahmenartigen Leiterelement 8 vollständig umgeben ist, wobei das Leiterelement 8 von den Einzelelektroden 7 elektrisch isoliert ist.

Über die Interfaceeinheit 6 und das Kabel 5 werden zur Durchführung einer Messung einer oder gleichzeitig oder sukzessive mehreren Einzelelektroden 7 mittels einer Signalquelle 9 je nach Betriebsart elektrische Spannungen und/oder elektrische Ströme zugeführt. Da die Patientin mittels einer nicht dargestellten, beispielsweise handgehaltenen Bezugselektrode über eine mit der Signalquelle 9 verbundene Leitung 10 auf ein Bezugspotential, z.B. -2,5 V, gelegt ist, werden somit elektrische Ströme und/oder Spannungen in den zu untersuchenden Gewebeabschnitt der Mamma 3 eingeprägt.

Diese Ströme und/oder Spannungen werden mittels einer Messschaltung 11 gemessen. Zu diesem Zweck sind (ist) die jeweils aktive(n) entsprechenden Einzelelektrode(n) 7, die auf Erdpotential liegen (liegt), über das Kabel 5 und die Interfaceeinheit 6 mit der Messschaltung 11 verbunden. Aus den gemessenen elektrischen Strömen und/oder Spannungen errechnet eine Auswerteeinheit 12 ein Impedanzbild, das auf einem Monitor 13 angezeigt wird.

Außerdem sind Potentialmittel 14 vorgesehen, die über die Interfaceeinheit 6 und das Kabel 5 mit dem Leiterelement 8 verbunden sind und dazu dienen, das Leiterelement 8 auf ein von dem der Einzelelektroden 7 abweichendes Potential, das vorzugsweise geringer als das Potential der Einzelelektroden 7 ist, zu legen.

Eine Steuereinheit 15, an die eine Tastatur 16 und ein weiteres Bedienelement, im Falle des beschriebenen Ausführungsbeispiels eine Mouse 17, angeschlossen sind, dient dazu, die Messanordnung zu steuern und ist dazu mit der Signalquelle 9, der Messschaltung 11, der Auswerteschaltung 12 und den Potentialmittels 14 über einen Steuerbus 18 verbunden. Die Bedienung der Messanordnung erfolgt mittels der Tastatur 16 und der Mouse 17 z.B. anhand von Bedienmenues, die auf dem Monitor 13 angezeigt werden.

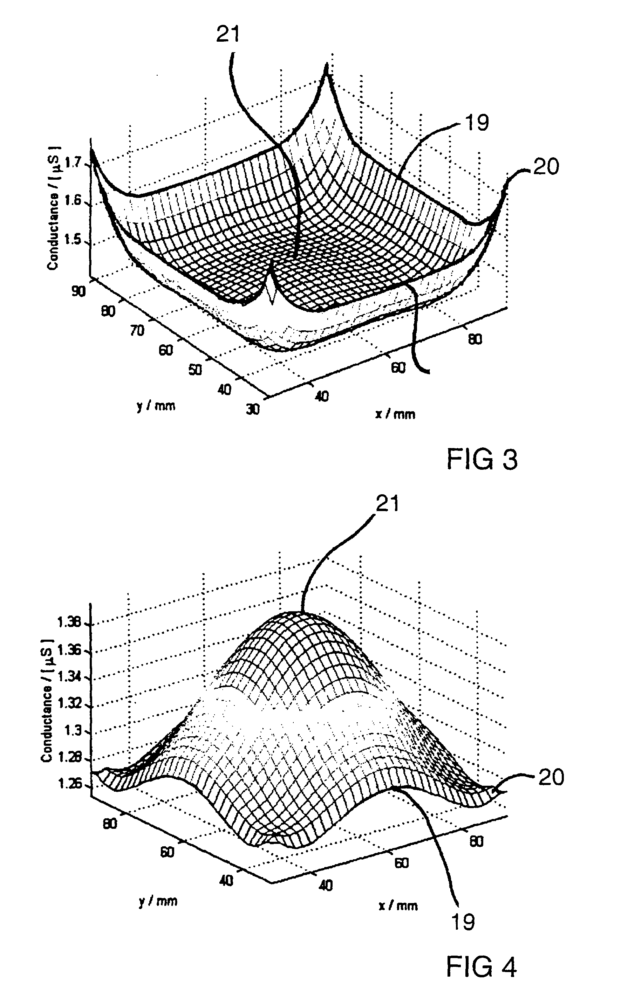

Wie eingangs erläutert, liegt im Falle des Standes der Technik das Leiterelement 8 auf dem gleichen Potential wie die Einzelelektroden 7. Ein für diesen Betriebszustand erzeugtes Impedanzbild ist in Fig. 3 dargestellt und weist an seinen Rändern 19 und Ecken 20, die den Rändern und Ecken der Elektrodenanordnung entsprechen, überhöhte Signale auf, während im Zentrum 21 des Impedanzbildes ein schwaches, nur schwer erkennbares Signal vorliegt, obwohl sich dort der zu detektierende Tumor 4 befindet.

Da im Falle der erfindungsgemäßen Messanordnung das Leiterelement 8 während einer Messung über die Leitung 5₁ und die Interfaceeinheit 6 des Kabels 5 mit den Potentialmitteln 14 in Verbindung steht und auf ein Potential unterhalb des Potentials der Einzelelektroden 7, beispielsweise auf ein Potential von -0,5 mV gelegt ist, werden die überhöhten Signale an den Rändern 19 und den Ecken 20 der Impedanzbilder unterdrückt, so dass das eigentlich zu detektierende Signal im Zentrum 21 des Impedanzbildes deutlich hervortritt und der Tumor 4 somit eindeutig feststellbar ist.

Wenn das Leiterelement 8 aus einem Material begrenzter Leitfähigkeit, also Widerstandsmaterial, gebildet ist und somit einen flächigen ohmschen Widerstand bildet, kann ein über die Breite b des Leiterelementes 8 sich änderndes Potential (Spannungs- oder Potential-Gradient) erzielt werden, indem während einer Messung der äußere Rand des Leiterelementes 8 über die Leitung 5₁ und die Interfaceeinheit 6 und der innere Rand des Leiterelements 8, wie strichliert angedeutet, über die Leitung 5₁ und die Interfaceeinheit 6 mit den Potentialmitteln 15 verbunden sind und von diesen auf unterschiedliche Potentiale, beispielsweise 0 mV am inneren Rand und -5 mV am äußeren Rand, gelegt werden, so dass sich über die Breite b des Leiterelements 8 ein z.B. linear abnehmender Spannungsverlauf einstellt, durch den eine besonders wirkungsvolle Unterdrückung unerwünschter Randeffekte im Impedanzbild und damit eine weiter verbesserte Bildqualität im Bereich der Ränder 19 des Impedanzbildes erreicht wird.

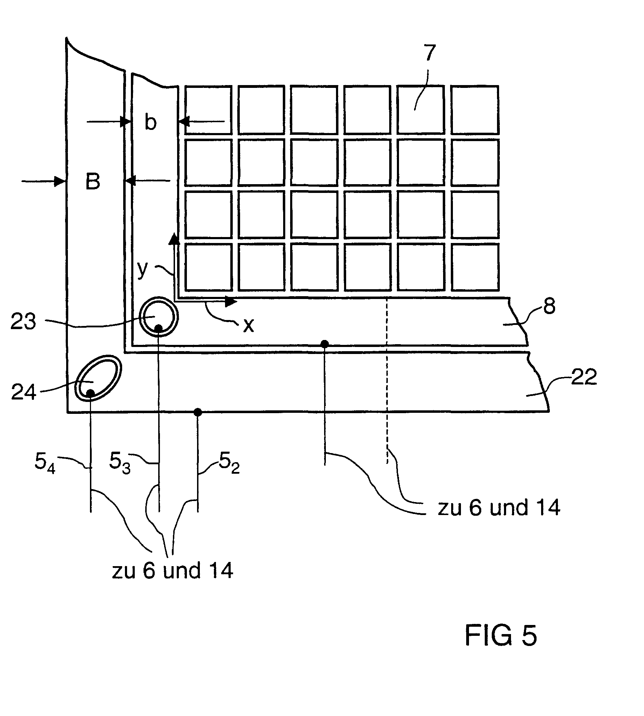

Eine nochmals verbesserte Bildqualität im Bereich der Ränder 19 des Impedanzbildes lässt sich gemäß Fig. 5 erreichen, wenn die ansonsten gemäß Fig. 2 aufgebaute Elektrodenanordnung ein das Leiterelement 8 vollständig umgebendes zweites rahmenartiges Leiterelement 22 aufweist, das von dem Leiterelement 8 und den Einzelelektroden 7 elektrisch isoliert ist und über eine Leitung 5₂ und die Interfaceeinheit 6 mit den Potentialmitteln 14 verbunden ist. Während einer Messung legen die Potentialmittel 14 das innere Leiterelement 8 beispielsweise auf ein Potential von -5 mV und das äußere Leiterelement 22 auf ein Potential von -15 mV, während die Einzelelektroden 7 auf einem Potential 0 mV liegen.

Wenn das innere Leiterelement 22, wie zuvor im Zusammenhang mit dem Leiterelement 8 beschrieben, aus Widerstandsmaterial gebildet ist, besteht in der schon beschriebenen Weise die Möglichkeit, den inneren Rand des Leiterelementes 8 auf ein Potential von 0 mV und den äußeren Rand auf ein Potential von - 5 mV zu legen.

Auch das eine gegenüber dem inneren Leiterelement 8 vergrößerte Breite B aufweisende äußere Leiterelement 22 kann in nicht dargestellter Weise aus Widerstandsmaterial gebildet sein. In diesem Fall beaufschlagen die Potentialmittel 14 den inneren Rand des Leiterelementes 22 mit einem Potential von - 5 mV oder wenig darüber und den äußeren Rand des äußeren Leiterelementes 22 mit einem Potential von -15 mV. Es entsteht dann wie im Falle des inneren Leiterelementes 8 ein Potentialgradient über der Breite B des Leiterelementes 22, der sich verbessernd auf die Bildqualität auswirkt.

Um die Bildqualitäten im Bereich der Ecken 20 der Impedanzbilder weiter zu verbessern, sind im Falle der Fig. 5 im Bereich der Ecken der Leiterelemente 8 und 22 lokale Leiterelemente 23 und 24 vorgesehen. Diese sind voneinander und von den Leiterelementen 8 und 22 sowie den Einzelelektroden 7 elektrisch isoliert und über Leitungen 5₃ und 5₄ und die Interfaceeinheit 6 mit den Potentialmitteln 14 verbunden. Diese legen die lokalen Leiterelemente 23 und 24 auf Potentiale, die unterhalb des Potentials des entsprechenden Leiterelements 8 bzw. 22 liegen. Beispielsweise legen die Potentialmittel 14 das lokale Leiterelement 23 auf ein Potential von - 7 mV und das lokale Leiterelement 24 auf ein Potential von - 20 mV.

Hierdurch werden trotz der Leiterelemente 8 und 22 eventuell noch vorhandene Signalüberhöhungen im Bereich der Ecken 20 der Impedanzbilder abgebaut.

Die Elektrodenanordnung kann mehr als zwei das Elektrodenarray umgebende Leiterelemente aufweisen, die unterschiedliche Breiten aufweisen und durch die Potentialmittel 14 mit unterschiedlichen Potentialen beaufschlagt werden.

Auch diese weiteren Leiterelemente können aus Material begrenzte Leitfähigkeit gebildet sein, so dass über ihre Breite abnehmende, zunehmende oder sonstige Potentialverläufe verwirklicht werden können.

Im Falle der Fig. 5 sind im Bereich sämtlicher Leiterelemente lokale Leiterelemente vorgesehen. Dies muss nicht notwendigerweise der Fall sein. Vielmehr kann sich die Anwesenheit lokaler Elemente auf einen Teil der Leiterelemente beschränken. Außerdem können, wie im Falle der Fig. 2, lokale Leiterelemente gänzlich fehlen.

Im Falle des Ausführungsbeispieles gemäß Fig. 5 sind die lokalen Leiterelemente im Bereich der Ecken der das Elektrodenarray umgebenden Leiterelemente vorgesehen. Die lokalen Leiterelemente können jedoch auch an anderen Stellen angeordnet sein. Auch können im Bereich der Ecken der das Elektrodenarray umgebenden Leiterelemente lokale Leiterelemente und zusätzlich zu diesen weitere lokale Leiterelemente vorgesehen sein.

Im Falle der beschriebenen Ausführungsbeispiele umgeben die Leiterelemente 8 und 22 die matrixförmige Anordnung der Einzelelektroden 7 des Elektrodenarrays vollständig. Es kann jedoch auch vorgesehen sein, dass Leiterelemente das Elektrodenarray nur bereichsweise umgeben.

Die im Falle der beschriebenen Ausführungsbeispiele vorgesehene Anzahl der das Elektrodenarray ganz oder bereichsweise umgebenden Leiterelemente sowie der lokalen Leiterelemente ist nur beispielhaft zu verstehen.

Die im Falle der Ausführungsbeispiele gezeigte quadratische Geometrie des Elektrodenarrays ist ebenso wie die quadratische Gestalt der Einzelelektroden, die rahmenförmige Gestalt der das Elektrodenarray umgebenden Leiterelemente sowie die kreisförmige bzw. elliptische Gestalt der lokalen Leiterelemente nur beispielhaft zu verstehen. In Abhängigkeit von dem jeweiligen Anwendungsfalls der Messanordnung sind auch abweichende Anordnungen und Geometrien möglich.

Die das Elektrodenarray ganz oder bereichsweise umgebenden Leiterelemente sowie die lokalen Leiterelemente müssen nicht sämtlich aktiv, d.h. auf ein von dem Potential der Einzelelektroden abweichendes Potential gelegt sein. Auch müssen nicht alle aktiven Leiterelemente auf ein Potential unterhalb des Potentials der Einzelelektroden gelegt sein; es kann vielmehr auch von Vorteil sein, einzelne Leiterelemente auf ein gegenüber den Einzelelektroden positives Potential zu legen.

Im Falle des beschriebenen Ausführungsbeispiels handelt es sich bei den Impedanzbildern um dreidimensionale Darstellungen des gemessenen Leitwerts (conductance) in Microsiemens (µS). Dabei verlaufen x- und y-Achsen parallel zu den Begrenzungskanten des Elektrodenarrays, weshalb x- und y-Achsen auch in Fig. 2 und Fig. 5 eingetragen sind. Der Leitwert ist auf der z-Achse aufgetragen.

Alternativ kann der jeweils gemessene Leitwert auch entsprechend einer Leitwert/Grauwertskala in einen Grauwert umgesetzt und somit ein zweidimensionales Schwarz-Weiß-Impedanzbild erzeugt und angezeigt werden.

Es kann vorgesehen sein, dass die Steuereinheit 15 vor der Durchführung der eigentlichen Messung zunächst ein Testbild erzeugt, dieses Testbild hinsichtlich der Signalwerte an den Ecken und Kanten auswertet und auf Basis dieser Auswertung die Potentialmittel 14 derart ansteuert, dass diese das Leiterelement bzw. die Leiterelemente mit geeigneten Potentialen beaufschlagt. Dabei erfolgt die automatische Auswahl der zu aktivierenden Leiterelemente sowie die automatische Einstellung der Potentiale unter Berücksichtigung von Art und Größe des zu untersuchenden Gewebeabschnittes, wobei diese Daten beispielsweise mittels der Tastatur 16 eingegeben werden.

Alternativ kann die Messanordnung derart betrieben werden, dass zunächst ein Testbild erzeugt wird und eine Bedienperson auf Grundlage dieses Testbildes mittels der Tastatur 16 bzw. der Mouse 17 die Potentiale des Leiterelementes bzw. der Leiterelemente einstellt, worauf ein weiteres Bild erzeugt wird. Sofern von der Bedienperson geeignete Potentiale gewählt wurden, handelt es sich bei diesem Bild dann um das gewünschte Impedanzbild. Andernfalls werden im Zuge eines iterativen Vorgehens die Potentiale so lange verändert, bis ein Impedanzbild ausreichender Qualität vorliegt.

Weiter kann vorgesehen sein, dass eine Bedienperson automatisch vorgenommene Einstellungen im Interesse einer optimalen Anpassung an den jeweiligen Untersuchungsfall manuell abwandelt.

Die beschriebenen Ausführungsbeispiele betreffen die Mammadiagnostik. Die erfindungsgemäße Messanordnung ist jedoch auch für andere Anwendungen geeignet, insbesondere auch zur Untersuchung nicht weiblicher Patienten.

## Patentansprüche

1. Messanordnung zur Untersuchung eines Gewebeabschnittes (3) eines Patienten, aufweisend ein Elektrodenarray, eine Signalquelle (9), eine Messschaltung (11) und Potentialmittel (14), wobei die Signalquelle (9) über wenigstens eine Elektrode (7) des Elektrodenarrays dem Patienten elektrische Ströme und/oder Spannungen einprägt, welche die Messschaltung (11) an mindestens einer Elektrode (7) des Elektrodenarrays misst, und wobei das Elektrodenarray zumindest bereichsweise von einem elektrischen Leiterelement (8, 22) umgeben ist,
**dadurch gekennzeichnet, dass** die Potentialmittel das Leiterelement (8, 22) mit einem vom Potential der Elektroden (7) des Elektrodenarrays abweichenden Potential beaufschlagen.

2. Messanordnung nach Anspruch 1, bei der das Leiterelement (8, 22) von den Potentialenmitteln (14) mit einem Potential beaufschlagt wird, das geringer als das Potential der Elektroden (7) ist.

3. Messanordnung nach Anspruch 1 oder 2, bei der das Leiterelement (8, 22) als Metallelement ausgebildet ist.

4. Messanordnung nach einem der Ansprüche 1 bis 3, bei der das Leiterelement (8, 22) als Kunststoffelement ausgebildet ist.

5. Messanordnung nach einem der Ansprüche 1 bis 4, bei der mindestens ein weiteres Leiterelement (22) vorgesehen ist.

6. Messanordnung nach Anspruch 5, bei der die Potentialmittel (14) die einzelnen Leiterelemente (8, 22) mit unterschiedlichen Potentialen beaufschlagen.

7. Messanordnung nach Anspruch 6, bei der die Elektrode (7), an welchen die Messschaltung (11) misst, auf einem Potential von 0 mV liegt und die Potentialmittel (14) ein inneres Leiterelement (8) mit einem Potential beaufschlagen, das kleiner als 0 mV ist und nicht unter -5 mV liegt, und ein äußeres Leiterelement (22) mit einem Potential von -15 mV beaufschlagen.

8. Messanordnung nach Anspruch 6 oder 7, bei der die Potentialmittel (14) die Potentiale der einzelnen Leiterelemente (8, 22) in Abhängigkeit von der Art und Größe des zu untersuchenden Gewebeabschnitts (3) einstellen.

9. Messanordnung nach einem der Ansprüche 5 bis 8, bei der die Leiterelemente (8, 22) geometrisch unterschiedlich ausgebildet sind.

10. Messanordnung nach Anspruch 9, bei der die Leiterelemente (8, 22) eine unterschiedliche Breite (b, B) aufweisen.

11. Messanordnung nach einem der Ansprüche 9 oder 10, deren Elektrodenarray mindestens ein lokales Leiterelement (23, 24) aufweist.

12. Messanordnung nach Anspruch 11, bei der ein lokales Leiterelement (23, 24) in einem Eckbereich des Elektrodenarrays angeordnet ist.

13. Messanordnung nach Anspruch 11 oder 12, bei der ein lokales Leiterelement (23, 24) von den Potentialmitteln (14) mit einem Potential beaufschlagt wird, das von dem Potential eines Leiterelementes (8, 22) und von dem Potential der Elektroden (7) abweicht.

14. Messanordnung nach einem der Ansprüche 11 bis 13, bei der die Potentialmittel (14) die Potentiale der einzelnen lokalen Leiterelemente (23, 24) in Abhängigkeit von der Art und Größe des zu untersuchenden Gewebeabschnitts (3) einstellen.

15. Messanordnung nach einem der Ansprüche 1 bis 14, bei der mindestens ein Leiterelement (8, 22) aus einem Material mit begrenzter Leitfähigkeit zur Ausbildung lokal unterschiedlicher Potentiale gebildet ist.

16. Messanordnung nach Anspruch 15, bei der ein Leiterelement (8, 22) aus einem Material mit begrenzter Leitfähigkeit als flächiger ohmscher Widerstand ausgebildet ist.

17. Messanordnung nach Anspruch 15 oder 16, bei der ein Leiterelement (8, 22) aus einem Material mit begrenzter Leitfähigkeit Ränder aufweist, an denen es die Potentialmittel (14) mit unterschiedlichen Potentialen beaufschlagen.

## Claims

1. A measuring arrangement for examining a tissue section (3) of a patient, having an electrode array, a signal source (9), a measuring circuit (11) and potential means (14), the signal source (9) subjecting the patient via at least one electrode (7) of the electrode array to electrical currents and/or voltages, which the measuring circuit (11) measures on at least one electrode (7) of the electrode array, and the electrode array being surrounded at least partially by an electrical conductor element (8,22),
**characterised in that**,
the potential means apply a potential that differs from the potential of the electrodes (7) of the electrode array.

2. The measuring arrangement as claimed in Claim 1, in which a potential that is less than the potential of the electrodes (7) is applied to the conductor element (8, 22) by the potential means (14).

3. The measuring arrangement as claimed in Claim 1 or 2, in which the conductor element (8, 22) is designed as a metal element.

4. The measuring arrangement as claimed in one of Claims 1 to 3, in which the conductor element (8, 22) is designed as a plastic element.

5. The measuring arrangement as claimed in one of Claims 1 to 4, in which at least one further conductor element (22) is provided.

6. The measuring arrangement as claimed in Claim 5, in which the potential means (14) apply different potentials to the individual conductor elements (8, 22).

7. The measuring arrangement as claimed in Claim 6, in which the electrode (7) on which the measuring circuit (11) measures is at a potential of 0 mV, and the potential means (14) apply a potential that is less than 0 mV and not below -5 mV to an inner conductor element (8), and they apply a potential of -15 mV to an outer conductor element (22).

8. The measuring arrangement as claimed in Claim 6 or 7, in which the potential means (14) adjust the potentials of the individual conductor elements (8, 22) as a function of the type and size of the tissue section (3) to be examined.

9. The measuring arrangement as claimed in one of Claims 5 to 8, in which the conductor elements (8, 22) are designed with differing geometries.

10. The measuring arrangement as claimed in Claim 9, in which the conductor elements (8, 22) have differing width (b,B).

11. The measuring arrangement as claimed in one of Claims 9 or 10, whose electrode array has at least one local conductor element (23, 24).

12. The measuring arrangement as claimed in Claim 11, in which the local conductor element (23, 24) is arranged in a corner region of the electrode array.

13. The measuring arrangement as claimed in Claim 11 or 12, in which a potential that differs from the potential of a conductor element (8, 22) and from the potential of the electrodes (7) is applied to a local conductor element (23, 24) by the potential means (14).

14. The measuring arrangement as claimed in one of Claims 11 to 13, in which the potential means (14) adjust the potentials of the individual local conductor elements (23, 24) as a function of the type and size of the tissue section (3) to be examined.

15. The measuring arrangement as claimed in one of Claims 1 to 14, in which at least one conductor element (8, 22) is made of a material with limited conductivity in order to form locally differing potentials.

16. The measuring arrangement as claims in Claim 15, in which a conductor element (8, 22) made of a material with limited conductivity, is designed as flat ohmic impedance.

17. The measuring arrangement as claimed in Claim 15 or 16, in which a conductor element (8, 22) made of a material with limited conductivity has edges on which the potential means (14) apply differing potentials to it.

## Revendications

1. Dispositif de mesure pour l'étude d'une partie (3) tissulaire d'un patient, comportant un réseau d'électrodes, une source (9) de signal, un circuit (11) de mesure et des moyens (14) d'application de potentiels, la source (9) de signal imprimant par l'intermédiaire d'au moins une électrode (7) du réseau d'électrodes au patient des courants et/ou des tensions électriques que le circuit (11) de mesure mesure sur au moins une électrode (7) du réseau d'électrodes et le réseau d'électrodes étant entouré au moins en partie d'un élément (8, 22) conducteur de l'électricité, **caractérisé en ce que** le moyen d'application de potentiels applique à l'élément (8, 22) conducteur un potentiel différent du potentiel de l'électrode (7) du réseau d'électrodes.

2. Dispositif de mesure suivant la revendication 1, dans lequel il est appliqué à l'élément (8, 22) conducteur par les moyens (14) d'application de potentiels un potentiel qui est plus bas que le potentiel de l'électrode (7).

3. Dispositif de mesure suivant la revendication 1 ou 2, dans lequel l'élément (8, 22) conducteur est constitué sous la forme d'un élément métallique.

4. Dispositif de mesure suivant l'une des revendications 1 à 3, dans lequel l'élément (8, 22) conducteur est constitué sous la forme d'un élément en matière plastique.

5. Dispositif de mesure suivant l'une des revendications 1 à 4, dans lequel il est prévu au moins un autre élément (22) conducteur.

6. Dispositif de mesure suivant la revendication 5, dans lequel les moyens (14) d'application de potentiels appliquent aux divers éléments (8, 22) conducteurs des potentiels différents.

7. Dispositif de mesure suivant la revendication 6, dans lequel l'électrode (7) sur laquelle le circuit (11) de mesure effectue la mesure est portée à un potentiel de 0 mV et les moyens (14) d'application de potentiels appliquent à un élément (8) conducteur intérieur un potentiel qui est plus bas que 0 mV et qui n'est pas inférieur à -5 mV et à un élément (22) conducteur extérieur un potentiel de -15 mV.

8. Dispositif de mesure suivant la revendication 6 ou 7, dans lequel les moyens 14 d'application de potentiels règlent les potentiels des divers éléments (8, 22) conducteurs en fonction de la nature et de la dimension de la partie (3) tissulaire à étudier.

9. Dispositif de mesure suivant l'une des revendications 5 à 8, dans lequel les éléments (8, 22) conducteurs sont constitués géométriquement d'une manière différente.

10. Dispositif de mesure suivant la revendication 9, dans lequel les éléments (8, 22) conducteurs ont une largeur (b, B) différente.

11. Dispositif de mesure suivant l'une des revendications 9 ou 10, dont le réseau d'électrodes a au moins un élément (23, 24) conducteur local.

12. Dispositif de mesure suivant la revendication 11, dans lequel un élément (23, 24) conducteur local est disposé dans une zone de coin du réseau d'électrodes.

13. Dispositif de mesure suivant la revendication 11 ou 12, dans lequel il est appliqué à un élément (23, 24) conducteur local par les moyens (14) d'application de potentiels un potentiel qui est différent du potentiel d'un élément (8, 22) conducteur et du potentiel de l'électrode (7).

14. Dispositif de mesure suivant l'une des revendications 11 à 13, dans lequel les moyens (14) d'application de potentiels règlent les potentiels des divers éléments (23, 24) conducteurs locaux en fonction de la nature et de la dimension de la partie (3) tissulaire à étudier.

15. Dispositif de mesure suivant l'une des revendications 1 à 14, dans lequel au moins un élément (8, 22) conducteur en un matériau de conductivité limitée est formé pour l'établissement de potentiels différents localement.

16. Dispositif de mesure suivant la revendication 15, dans lequel un élément (8, 22) conducteur est constitué en un matériau de conductivité limitée sous la forme d'une résistance ohmique plate.

17. Dispositif de mesure suivant la revendication 15 ou 16, dans lequel un élément (8, 22) conducteur en un matériau de conductivité limitée a des bords sur lesquels les moyens (14) d'application de potentiels appliquent des potentiels différents.
